(19) Europäisches Patentamt
European Patent Office
Office européen des brevets

(11) EP 2 540 317 A1

(12) EUROPEAN PATENT APPLICATION
published in accordance with Art. 153(4) EPC

(43) Date of publication:
**02.01.2013 Bulletin 2013/01**

(21) Application number: **11744791.2**

(22) Date of filing: **21.02.2011**

(51) Int Cl.:
***A61K 47/34*** (2006.01) ***A61K 9/16*** (2006.01)
***A61K 31/403*** (2006.01) ***A61K 31/4745*** (2006.01)
***A61K 47/12*** (2006.01) ***A61K 47/14*** (2006.01)
***A61K 47/38*** (2006.01) ***A61P 7/02*** (2006.01)

(86) International application number:
**PCT/JP2011/053643**

(87) International publication number:
**WO 2011/102505 (25.08.2011 Gazette 2011/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.02.2010 JP 2010035883**

(71) Applicant: **Daiichi Sankyo Company, Limited**
**Tokyo 103-8426 (JP)**

(72) Inventors:
- **KANAMARU, Taro**
  **Tokyo 140-8710 (JP)**
- **TAJIRI, Shinichiro**
  **Tokyo 140-8710 (JP)**

(74) Representative: **Fairbairn, Angus Chisholm**
**Marks & Clerk LLP**
**90 Long Acre**
**London**
**WC2E 9RA (GB)**

# (54) SUSTAINED-RELEASE SOLID PREPARATION FOR ORAL USE

(57) It is intended to avoid dose dumping of a drug and improve the dissolution properties of the drug in the lower gastrointestinal tract, and thereby provide a sustained-release pellet preparation for oral administration that reliably exhibits its main pharmacological effect when orally administered once or twice a day. The present invention provides a sustained-release preparation obtained by mixing of (A) a pharmacologically active drug, (B) hydroxypropyl methylcellulose acetate succinate, (C) a plasticizer, and (D) polyethylene glycol followed by extrusion granulation.

EP 2 540 317 A1

## Description

### Technical Field

The present invention relates to a matrix pellet preparation that reliably exhibits its main pharmacological effect when orally administered once or twice a day.

### Background Art

Sustained-release preparations for the adjustment of blood concentrations of drugs are highly useful in terms of separation between the main pharmacological effect and adverse reaction, improvement in compliance (e.g., the number of doses reduced by improvement in prolonged efficacy), medical economy, etc. In this regard, some techniques have been reported for sustained-release preparations. Meanwhile, since compounds exhibiting the main pharmacological effect have diverse chemical properties, some sustained release techniques, albeit still insufficient, adaptable to the diverse chemical properties of these compounds have been reported (see e.g., Patent Documents 1 and 2).

The properties of a drug itself can be classified broadly into neutral, acidic, and basic properties. Among others, solubility (degree of solubility) in water differs greatly between compounds. Low water-soluble compounds have many disadvantages in the design of preparations to improvedissolution properties. Acidic drugs refer to acidic compounds that are acidic in the free form (whose acidic group does not constitute a salt such as an alkali- or amine-addition salt). Acidic drugs are disadvantageously low soluble in acidic solutions, for example, in the upper gastrointestinal tract such as the stomach. A salt (alkali- or amine-addition salt) of an acidic compound disadvantageously becomes a low soluble free acid in an acidic solution. Alternatively, basic drugs refer to basic compounds that are basic in the free form (whose basic group does not constitute a salt such as an acid-addition salt) and are known to exhibit favorable solubility in strongly acidic aqueous solutions, but exhibit reduced solubility in a neutral aqueous solutions such as a neutral buffer. Specifically, basic drugs, when orally administered, exhibit favorable solubility in the stomach, which is acidic. Their solubility, however, is greatly reduced in the lower gastrointestinal tract such as the large intestine, which is neutral with little water, probably leading to a reduced absorption rate of the drug.

For example, a challenge for the design of sustained-release preparations for oral administration containing a basic drug is dose dumping of the drug when the preparation collapses due to mechanical stress resulting from the presence of food in the acidic environment of the upper gastrointestinal tract exhibiting high water-solubility, gastrointestinal motility, and so on. Furthermore, preparation strength may be enhanced by, for example, an increased amount of a sustained-release agent in order to avoid dose dumping of the drug. In such a case, still, the challenge for a sustained-release preparation containing a basic drug whose water solubility is reduced in the neutral region is to improve the dissolution properties of the preparation in the lower gastrointestinal tract and maintain drug absorption. No previous technique for sustained-release preparations containing a basic drug can simultaneously achieve, at satisfactory levels, avoidance of dose dumping of the drug in an acidic environment such as the upper gastrointestinal tract and prolonged dissolution in the lower gastrointestinal tract, which is a neutral environment.

### Citation List

### Patent Document

Patent Document 1: National Publication of International Patent Application No. 2006/507216
Patent Document 2: National Publication of International Patent Application No. 2004/518676

### Summary of Invention

### Technical Problem

An object of the present invention is to avoid dose dumping of a drug caused by mechanical stress resulting from gastrointestinal motility in the presence of food in the acidic environment of the upper gastrointestinal tract, particularly, the stomach, and to improve the dissolution properties of the drug in the lower gastrointestinal tract, which is the neutral region, and thereby provide a sustained-release preparation for oral administration containing, as a principal pharmaceutically active ingredient, a drug that reliably exhibits its main pharmacological effect when orally administered once or twice a day.

Solution to Problem

As a result of conducting studies on the formulation of sustained-release preparations for oral administration, the present inventors have found that a sustained-release matrix pellet preparation containing a pharmacologically active drug, a pH-dependent polymer base, a plasticizer, and a low-melting-point oil or fat can avoid dose dumping of the drug under an acidic environment and can be improved in its dissolution properties in the neutral region. Based on this finding, the present invention has been completed.
Specifically, the present invention relates to the following (1) to (44):

(1) A sustained-release matrix pellet preparation obtained by extrusion granulation, containing the following components (A) to (D):

(A) a pharmacologically active drug;
(B) a pH-dependent polymer base;
(C) a plasticizer; and
(D) a low-melting-point oil or fat.

(2) The sustained-release matrix pellet preparation according to (1), wherein the pH-dependent polymer base (B) is an enteric coating base.
(3) The sustained-release matrix pellet preparation according to (2), wherein the enteric coating base is hydroxypropyl methylcellulose acetate succinate.
(4) The sustained-release solid preparation according to any one of (1) to (3), wherein the content of the pH-dependent polymer base (B) in the matrix pellets preparation is in the range of 5 to 90% by weight.
(5) The sustained-release matrix pellet preparation according to any one of (1) to (4), wherein the plasticizer (C) is citric acid tri(C1-C6 alkyl) ester or glycerin mono-organic acid ester.
(6) The sustained-release matrix pellet preparation according to any one of (1) to (4), wherein the plasticizer (C) is triethyl citrate or glycerin monoacetic acid ester.
(7) The sustained-release solid preparation according to any one of (1) to (6), wherein the content of the plasticizer (C) in the matrix pellet preparation is in the range of 1 to 30% by weight.
(8) The sustained-release matrix pellet preparation according to any one of (1) to (7), wherein the low-melting-point oil or fat (D) is glycerin monostearate or polyethylene glycol.
(9) The sustained-release matrix pellet preparation according to any one of (1) to (7), wherein the low-melting-point oil or fat (D) is polyethylene glycol.
(10) The matrix pellet preparation according to (8) or (9), wherein the polyethylene glycol is polyethylene glycol having an average molecular weight in the range of 4000 to 12000.
(11) The sustained-release matrix pellet preparation according to (8) or (9), wherein the polyethylene glycol is polyethylene glycol having an average molecular weight in the range of 7000 to 9000.
(12) The sustained-release solid preparation according to any one of (1) to (11), wherein the content of the low-melting-point oil or fat (D) in the matrix pellets preparation is in the range of 0.1 to 20% by weight.
(13) The sustained-release matrix pellet preparation according to any one of (1) to (12), further containing one or two or more components selected from a binder and an organic acid.
(14) The sustained-release matrix pellet preparation according to (13), wherein the binder is a hydrophilic gel-forming polymer material.
(15) The sustained-release matrix pellet preparation according to (14), wherein the hydrophilic gel-forming polymer material is hydroxypropyl cellulose.
(16) The sustained-release matrix pellet preparation according to any one of (13) to (15), wherein the organic acid is fumaric acid or alginic acid.
(17) The sustained-release matrix pellet preparation according to any one of (13) to (15), wherein the organic acid is fumaric acid.
(18) The sustained-release matrix pellet preparation according to any one of (1) to (17), wherein the pharmacologically active drug (A) exhibits the following degree of solubility:

(degree of solubility in the neutral state) / (degree of solubility in the acidic state) in the range of 0.00001 to 0.6.

(19) The sustained-release matrix pellet preparation according to any one of (1) to (17), wherein the pharmacologically active drug (A) exhibits the following degree of solubility:

(degree of solubility in the neutral state) / (degree of solubility in the acidic state) in the range of 0.001 to 0.5.

(20) The sustained-release matrix pellet preparation according to any one of (1) to (17), wherein the pharmacologically active drug (A) exhibits the following degree of solubility:

the lowest degree of solubility in the neutral state (in the range of 7.5 > pH > 5) of 3 mg/ml or lower.

(21) The sustained-release matrix pellet preparation according to any one of (1) to (17), wherein the pharmacologically active drug (A) exhibits the following degree of solubility:

the lowest degree of solubility in the neutral state (in the range of 7.5 > pH > 5) of 0.5 mg/ml or lower.

(22) The sustained-release matrix pellet preparation according to any one of (1) to (21), wherein the pharmacologically active drug (A) is a basic drug.
(23) The sustained-release matrix pellet preparation according to any one of (1) to (17), wherein the pharmacologically active drug (A) is a compound selected from the group consisting of the following:

(±)-1-(carbazol-4-yloxy)-3-[[2-(o-methoxyphenoxy)ethyl]amino]-2-propanol;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide; and
$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide

or a pharmacologically acceptable salt thereof, or a hydrate thereof.
(24) The sustained-release solid preparation according to any one of (1) to (23), wherein the content of the pharmacologically active drug (A) in the matrix pellet preparation is in the range of 0.1 to 50% by weight.
(25) The sustained-release matrix pellet preparation according to any one of (1) to (24), wherein the matrix pellet preparation is matrix pellets.
(26) The sustained-release matrix pellet preparation according to any one of (1) to (25), wherein when the preparation is subjected to a dissolution test by the paddle method at a rotation rate of 50 rpm at 37 ± 0.5°C in a 0.01 N aqueous hydrochloric acid solution (900 mL), the preparation exhibits an average percentage dissolution of the pharmacologically active drug of 60% or lower in 4 hours after the start of the dissolution test.
(27) A method for producing a sustained-release matrix pellet preparation for oral administration according to any one of (1) to (26), comprising the following steps (E) to (H):

(E) mixing a pharmacologically active drug, a pH-dependent polymer base, and an oil or fat to prepare a [mixture];
(F) adding and stirring a plasticizer, an aqueous binder solution, and distilled water to prepare a [dispersion];
(G): producing a [kneaded product] from the [powder mixture] and the [dispersion]; and
(H) extrusion-granulating the [kneaded product] to produce [pellets].

(28) A sustained-release preparation obtained by mixing of (A) a pharmacologically active drug, (B) hydroxypropyl methylcellulose acetate succinate, (C) a plasticizer, and (D) polyethylene glycol followed by extrusion granulation.
(29) The preparation according to (28), wherein the content of the component (B) in the preparation is 50 to 90% by weight.
(30) The preparation according to (28), wherein the content of the component (B) in the preparation is 55 to 80% by weight.
(31) The preparation according to any one of (28) to (30), wherein the component (C) is triethyl citrate.
(32) The preparation according to any one of (28) to (31), wherein the content of the component (C) in the preparation is 5 to 30% by weight.
(33) The preparation according to any one of (28) to (32), wherein the component (D) is polyethylene glycol having an average molecular weight in the range of 4000 to 12000.
(34) The preparation according to any one of (28) to (32), wherein the component (D) is polyethylene glycol having an average molecular weight in the range of 7000 to 10500.
(35) The preparation according to any one of (28) to (32), wherein the component (D) is polyethylene glycol 6000.
(36) The preparation according to any one of (28) to (35), wherein the content of the component (D) in the preparation is 1 to 10% by weight.
(37) The preparation according to any one of (28) to (35), wherein the content of the component (D) in the preparation is 2 to 8% by weight.
(38) The preparation according to any one of (28) to (37), further containing a binder and/or an organic acid.
(39) The preparation according to (38), wherein the binder is hydroxypropyl cellulose.

(40) The preparation according to (38) or (39), wherein the organic acid is fumaric acid or alginic acid.
(41) The preparation according to (38) or (39), wherein the organic acid is fumaric acid.
(42) The preparation according to any one of (28) to (41), wherein the component (A) is a basic drug.
(43) The preparation according to any one of (28) to (41), wherein the component (A) is a compound selected from the group consisting of

(±)-1-(carbazol-4-yloxy)-3-[[2-(o-methoxyphenoxy)ethyl]amino]-2-propanol,
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino)cyclohexyl)ethanediamide, and
$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide

or a pharmacologically acceptable salt thereof, or a hydrate thereof.
(44) The preparation according to any one of (28) to (43), wherein the content of the component (A) in the preparation is 0.1 to 50% by weight.

Advantageous Effects of the Invention

The present invention provides a sustained-release matrix pellet preparation for oral administration containing a pharmacologically active drug typified by compound (1). The sustained-release matrix pellet preparation of the present invention has favorable strength that prevents dose dumping in an acidic solution, and has favorable dissolution properties in a neutral solution. Thus, the sustained-release pharmaceutical composition of the present invention is effective for maintaining a prolonged dissolution of the pharmacologically active drug contained therein from the duodenum through the small intestine to the lower gastrointestinal tract.

Brief Description of the Drawings

[Figure 1] Figure 1 is a diagram showing the dissolution behaviors (the paddle method, paddle rotation rate: 50 rpm, test medium quantity: 900 mL) in 0.01 N hydrochloric acid of 4 formulations produced by an extrusion granulation method, for the formulations examined in Table 1 of Example 1.
[Figure 2] Figure 2 is a diagram showing the dissolution behaviors (the paddle method, paddle rotation rate: 50 rpm, test medium quantity: 900 mL) in 0.01 N hydrochloric acid of 4 formulations produced by an extrusion granulation method, for the formulations examined in Table 3 of Example 2.
[Figure 3] Figure 3 is a diagram showing the dissolution behaviors (the paddle method; paddle rotation rate: 50 rpm, test medium: 4-fold diluted solution of phosphate buffer, pH 6.8 (USP), test medium quantity: 900 mL) of glycerin monostearate-supplemented pellets (formulation 9) of Example 3 after storage at 40°C, 50°C, or 60°C (in a tightly stoppered vial) for 1 day.
[Figure 4] Figure 4 is a diagram showing the dissolution behaviors (the paddle method; paddle rotation rate: 50 rpm, test medium: 4-fold diluted solution of phosphate buffer, pH 6.8 (USP), test medium quantity: 900 mL) of a polyethylene glycol-supplemented formulation (formulation 10) of Example 3 after storage at 40°C, 50°C, or 60°C (in a tightly stoppered vial) for 1 week.
[Figure 5] Figure 5 is a diagram showing time-dependent change in the dissolution rate of a drug in a dissolution test in a neutral solution (the paddle method; paddle rotation rate: 50 rpm, test medium: 4-fold diluted solution of phosphate buffer, pH 6.8 (USP), test medium quantity: 900 mL) for formulation 11 described in Table 6 of Example 5.
[Figure 6] Figure 6 is a diagram showing time-dependent change in the dissolution rate of a drug in a dissolution test in a neutral solution (the paddle method; paddle rotation rate: 50 rpm, test medium: 4-fold diluted solution of phosphate buffer, pH 6.8 (USP), test medium quantity: 900 mL) for formulation 12 described in Table 7 of Example 6.

Description of Embodiments

Hereinafter, the present invention will be described in detail.
In the present specification, "acidic solution" means an acidic dissolution test medium used for evaluation of dissolution properties in the upper gastrointestinal tract such as the stomach. Non-limiting examples of the acidic dissolution test medium can include: the JP 1st dissolution test fluid described in the Japanese Pharmacopoeia; and USP 0.1 N hydrochloric acid, 0.01 N hydrochloric acid, and Simulated Gastric Fluid without Enzyme described in the United States Pharmacopoeia.

In the present specification, "neutral solution" means a neutral dissolution test medium used for evaluation of drug dissolution properties in the small intestine, the large intestine, or the like. Non-limiting examples of the neutral dissolution test medium can include dissolution test media (pH 6.8) such as: the JP 2nd dissolution test fluid and phosphate buffer (pH 6.8) described in the Japanese Pharmacopoeia; USP Phosphate Buffer (pH 6.8) and Simulated Intestinal Fluid without Enzyme described in the United States Pharmacopoeia; and Phosphate Buffer Solution (pH 6.8) described in the European Pharmacopoeia.

The aforementioned dissolution test medium is prepared through methods described in the corresponding pharmacopoeia or the like of each country. When the employed dissolution test medium is a buffer solution, variation of the pH of the test medium is preferably within ±0.05 of the pH defined for each dissolution medium.

Examples of the paddle method using an acidic dissolution medium for the evaluation of dissolution properties of the sustained-release matrix pellet preparation of the present invention in the upper gastrointestinal tract can include a method in which a dissolution test is conducted by the paddle method at rotation rates of 50 rpm and 200 rpm at 37 ± 0.5°C for 2 hours in 0.01 N hydrochloric acid (900 mL). As described above, when the pharmacologically active drug in the preparation is a basic drug, dose dumping of the drug becomes a problem because the preparation collapses due to mechanical stress resulting from the presence of food in the acidic environment of the upper gastrointestinal tract exhibiting high water-solubility, gastrointestinal motility, and so on. Thus, the average percentage dissolution of the pharmacologically active drug in the dissolution test medium is preferably a value that allows preparation strength to be maintained and the dissolution rate to be kept within a predetermined range at the rotation rates of 200 rpm and/or 50 rpm in the paddle method. Moreover, when the preparation is subjected to the dissolution test method for 2 hours, the difference in average percentage dissolution (value at the rotation rate of 200 rpm in the paddle method - value at the rotation rate of 50 rpm in the paddle method) of the pharmacologically active drug in the dissolution test medium is preferably 15% or lower, more preferably 10% or lower, particularly preferably 5% or lower. Furthermore, the average percentage dissolution ratio (value at the rotation rate of 200 rpm in the paddle method / value at the rotation rate of 50 rpm in the paddle method) of the pharmacologically active drug in the dissolution test medium after 2 hours is preferably 2.0 or lower, more preferably 1.5 or lower, particularly preferably 1.3 or lower.

USP Apparatus 3 (Bio-Dis method), which is a dissolution test method under conditions close to the environment of the human gastrointestinal tract, may be used for the dissolution test.
The concentration of the drug in a solution can be measured using conditions (test medium, shaking rate, and measurement time) shown in Examples described later. In the dissolution test, the average percentage dissolution and dissolution time of the pharmacologically active drug in the dissolution test medium can be calculated using the UV method or the like.

As used herein, "average percentage dissolution" refers to the average of percentage dissolution values obtained from at least 2, preferably 6, more preferably 12 solid preparation samples for each type of solid preparation.

In the present specification, the "pharmacologically active drug" is preferably a relatively low water-soluble drug that exhibits the main pharmacological effect of the formulation of the preparation. A neutral compound of the pharmacologically active drug means a compound that does not have a group dissociable by ionization in the acidic or basic state in its molecule. Moreover, an acidic compound means a drug having an acidic group typified by a carboxy group, a phenolic hydroxy group, a phosphoric acid group, a sulfonic acid group, a tetrazolyl group, or the like. Furthermore, a basic drug means a drug having a basic nitrogen atom typified by an amino group, a piperidinyl group, a piperazinyl group, or the like in its molecule. In the present invention, particularly, a basic drug is preferred. The basic drug has physicochemical properties in which the degree of solubility is lower in the neutral state ($7.5 > pH > 5$) in the small intestine or the large intestine than that in the acidic state ($pH \leq 2$).
As described above, the basic drug refers to a drug having a degree of solubility that is lower in the neutral state than in the acidic state. Non-limiting examples of the rate of this reduction in the degree of solubility in the neutral state can include the following ranges:

preferably, (degree of solubility in the neutral state) /(degree of solubility in the acidic state) in the range of 0.00001 to 0.6;
more preferably, (degree of solubility in the neutral state) / (degree of solubility in the acidic state) in the range of 0.001 to 0.5; and
even more preferably, (degree of solubility in the neutral state) / (degree of solubility in the acidic state) in the range of 0.01 to 0.1.

In the present specification, the "basic drug" preferably has a degree of solubility in the range of 1 to 500 mg/ml in the acidic region (the JP 1st dissolution test fluid; pH 1.2, 20 ± 5°C) and a degree of solubility in the range of 0.01 to 3000 μg/ml in the neutral region (the JP 2nd dissolution test fluid; pH 6.8, 20 ± 5°C).
More preferred is a basic drug having a degree of solubility in the range of 1 to 500 mg/ml in the acidic region (the JP 1st dissolution test fluid; pH 1.2, 20 ± 5°C) and a degree of solubility in the range of 10 to 500 mg/ml in the neutral region

(the JP 2nd dissolution test fluid; pH 6.8, 20 ± 5°C). Moreover, the absolute value of the degree of solubility in the drug is preferably the lowest degree of solubility reduced to 3 mg/ml or lower, more preferably 1 mg/ml or lower, even more preferably 0.5 mg/ml or lower, in the neutral state (in the range of 7.5 > pH > 5).

Specific examples of the "pharmacologically active drug" can include anticoagulant agents.

The anticoagulant agent is preferably an activated blood coagulation factor X (FXa) inhibitor. Specific examples of the FXa inhibitor can include the following (a) to (1) :

(a) Darexaban Maleate (tanexaban) (N-[2-hydroxy-6-methoxybenzamido]phenyl]-4-(4-methyl-1,4-diazepan-l-yl) benzamide) [See PFSB/ELD No. 1111-1 (November 11, 2010); Pre-publication copy, Proposed INN: List 101; Research and development pipeline. Yamanouchi Pharmaceutical Co Ltd. Company World Wide Web site, 11 Feb 2004];
(b) rivaroxaban (5-chloro-N-({(5S)-2-oxo-3-[4-(3-oxo-4-morpholinyl)phenyl]-1,3-oxazolidin-5-yl}methyl)-2-thiophenecarboxamide) [See WFO Drug Information, Vol. 18, No. 3, 2004, page 260; Susanne R, et al, J. Med. Chem., 2005, 48, 5900-5908; D. Kubitza et al, Multiple dose escalation study investigating the pharmacodynamics, safety, and pharmacokinetic of Bay59-7939, an oral, direct Factor Xa inhibitor, in healthy male subjects. Blood, 2003, 102; Abstract 3004];
(c) apixaban (1-{4-methoxyphenyl)-7-oxo-6-[4-(2-oxopiperidin-1-yl)phenyl]-4,5,6,7-tetrahydro-1H-pyrazolo[3,4-c] pyridine-3-carboxamide) [See WFO Drug Information, Vol. 20, No. 1, 2006, page 38; Pinto DJP, Orwat MJ, Lam PYS, et al, Discovery of 1-(4-Methoxyphenyl)-7-oxo-6-(4-(2-oxopiperidin-1-yl)phenyl)-4,5,6,7-tetrahydro-1H-pyrazolo[3,4-c]pyridine-3-carboxamide (Apixaban, BMS-562247), a highly potent, selective, efficacious, and orally bioavailable inhibitor of blood coagulation factor Xa, J. Med. Chem., 50 (22), 5339-56, 2007];
(d) Betrixaban (N-(5-chloropyridin-2-yl)-2-[4-(N,N-dimethylcarbamimidoyl)benzamido]-5-methoxybenzamide) [See WFO Drug Information, Vol. 22, No. 3, 2008, page 226-227; Zhang P, Huang W, Zhu BY, et al., Discovery of betrixaban (PRT054021), N-(5-chloropyridin-2-yl)-2-(4-(N,N-dimethylcarbamimidoyl)benzamido)-5-methoxybenzamide, a highly potent, selective, and orally efficacious factor Xa inhibitor, Bioorg Med. Chem. Lett. 19 (8), 2179-85, 2009] ;
(e) AX-1826, [S. Takehana et al. Japanese Journal of Pharmacology 2000, 82 (Suppl. 1), 213P; T. Kayahara et al. Japanese Journal of Pharmacology 2000, 82 (Suppl. 1), 213P];
(f) HMR-2906, [XVIIth Congress of the International Society for Thrombosis and Haemostasis, Washington D. C., USA, 14-21 Aug 1999; Generating greater value from our products and pipeline. Aventis SA Company Presentation, 05 Feb 2004];
(g) Otamixaban (methyl (2R, 3R)-2-(3-carbamimidoylbenzyl)-3-[[4-(1-oxidopyridin-4-yl)benzoyl]amino]butanoate) [See WFO Drug Information, Vol. 16, No. 3, 2002, page 257];
(h) BIBT-986 (prodrug: BIBT-1011) [American Chemical Society-226th National Meeting, New York City, NY, USA, 2003];
(i) DPC-602, [J. R. Pruitt et al. J. Med. Chem. 2003, 46, 5298-5313];
(j) LY517717 (N-[(1R)-2-[4-(1-methyl-4-piperidinyl)-1-piperazinyl]-2-oxo-1-phenylethyl]-1H-indole-6-carboxamide) [See S. Young, Medicinal Chemistry-12th RSC-SCI Symposium, 7-10 September 2003, Cambridge, UK; M. Wiley et al. 228th ACS National Meeting, Philadelphia, August 22-26, 2004, MEDI-252 & 254];
(k) N1-(5-chloropyridin-2-yl)-$N^2$-[(1S,2R,4S)-2-{[C(5-methyl-4,5,6,7-tetrahydrothiazolo[5,4-c]pyridin-2-yl)carbonyl] amino}-4-([1,3,4]oxadiazol-2-yl)cyclohexyl]ethanediamide or a pharmacologically acceptable salt thereof, or a hydrate thereof [see WO 2004/058715]; and
(l) $N^1$-(5-chloropyridin-2-yl)-$N^2$-((1S,2R,4S)-4-[(dimethylamino)carbonyl]-2-{[(5-methyl-4,5,6,7-tetrahydrothiazolo [5,4-c]pyridin-2-yl)carbonyl]amino)cyclohexyl)ethanediamide or a pharmacologically acceptable salt thereof, or a hydrate thereof [see WO 03/000657; WO 03/000680; and WO 03/016302].

The aforementioned activated blood coagulation factor X (FXa) inhibitor is more preferably a compound represented by the following formula (1) [hereinafter, also abbreviated to compound (1)]:

[Formula 1]

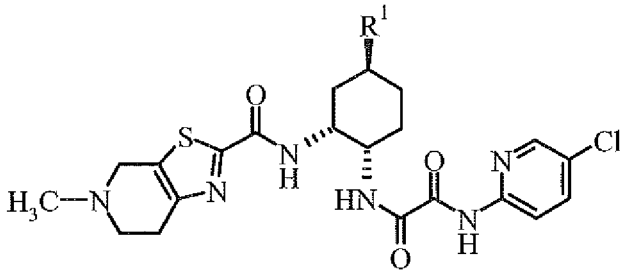

(1)

wherein $R^1$ represents an N,N-dimethylcarbamoyl group or a [1,3,4]oxadiazol-2-yl group. Compound (1) may be the free form (free base) or a pharmacologically acceptable salt thereof, or a hydrate thereof.
Examples of the salt of the compound represented by formula (1) include hydrochloride, sulfate, hydrobromide, hydroiodide, phosphate, nitrate, benzoate, methanesulfonate, 2-hydroxyethanesulfonate, p-toluenesulfonate, acetate, propionate, oxalate, malonate, succinate, glutarate, adipate, tartrate, maleate, fumarate, malate, and mandelate.
The salt of the compound represented by formula (1) is preferably maleic acid, hydrochloride, methanesulfonate, or p-toluenesulfonate, particularly preferably maleate or p-toluenesulfonate.
Preferable examples of the compound represented by formula (1) can include the following:

$N^1$-(5-chloropyridin-2-yl)-$N^2$-[(1S,2R,4S)-2-{[(5-methy-1-4,5,6,7-tetrahydrothiazolo[5,4-c]pyridin-2-yl)carbonyl]amino}-4-([1,3,4]oxadiazol-2-yl)cyclohexyl]ethanediamide monomaleate;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide hydrochloride;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate; and
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate monohydrate.

Among these preferable compounds, particularly preferred are

$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide monomaleate (1a); and
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate monohydrate (1b)

represented by the following formula (1a) [hereinafter, also abbreviated to compound (1a)] and formula (1b) [hereinafter, also abbreviated to compound (1b)], respectively:
[Formula 2]

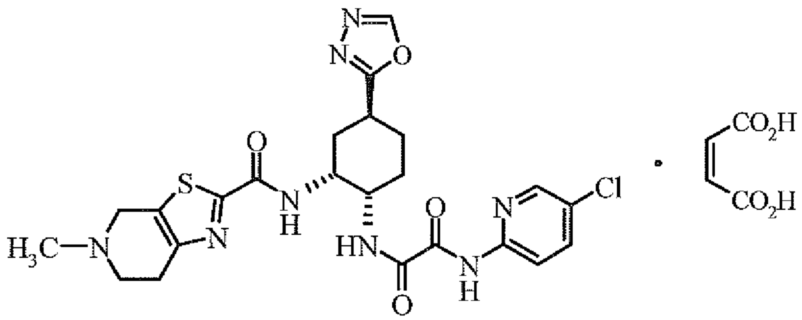

(1a)

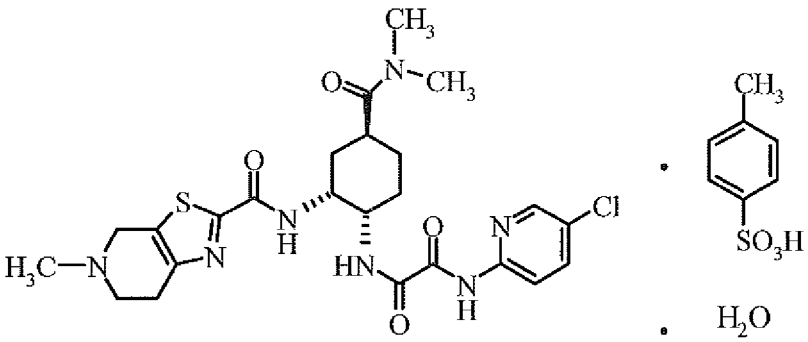

(1b)

These compounds (1) can be produced by a method described in documents or a method equivalent thereto (WO 2003-000657; WO 2003-000680; WO 2003-016302; and WO 2004-058715).

The free base (free form) of compound (1) means the salt (acid-addition salt) and/or the hydrate formed with compound (1) except for "acid" in the acid-addition salt or "water" in the hydrate. For example, the free bases (free forms) of compound (1a) and compound (1b) mean $N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide (1a-1) and $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl) carbonyl]amino}cyclohexyl)ethanediamide (1b-1)

represented by the following formula (1a-1) and formula (1b-1), respectively:

[Formula 3]

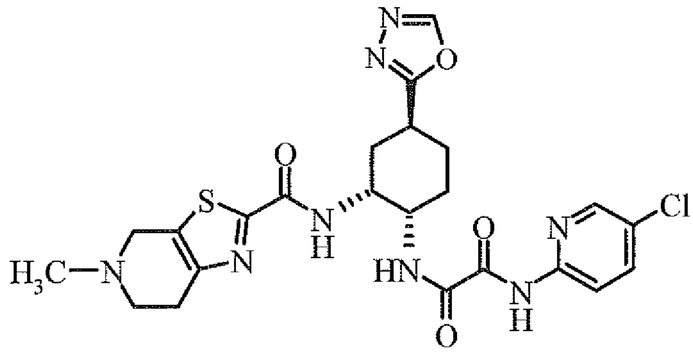

(1a-1)

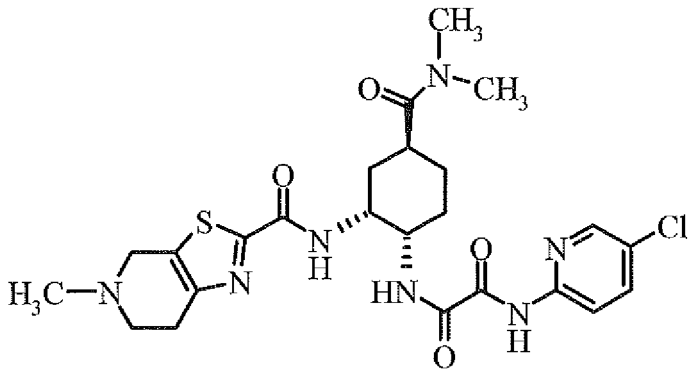

(1b-1)

Moreover, preferable examples of the pharmacologically active drug (A) of the present invention can include (+) -1-(carbazol-4-yloxy)-3-[[2-(o-methoxyphenoxy)ethyl]amino]-2-propanol (3) (CAS No.: 72956-09-3) represented by the following formula (2) [hereinafter, also abbreviated to compound (2)]:

[Formula 4]

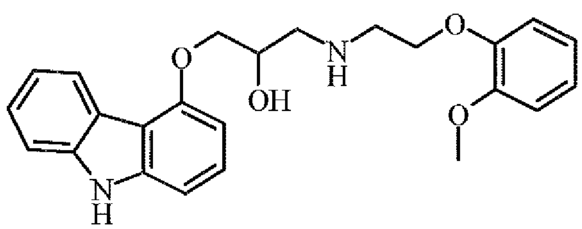

(2)

or a pharmacologically acceptable salt thereof, or a hydrate thereof.

Examples of the "pH-dependent polymer base (B)" according to the present invention can include polymer bases that exhibit pH-dependent dissolution properties used in the pharmaceutical field. The "pH-dependent polymer base" can further encompass enteric coating bases and gastric soluble bases. An enteric coating base is preferred. The preferred enteric coating base is hardly soluble under a pH environment such as the stomach and is gradually dissolved under a neutral pH environment such as the small intestine or the large intestine, which is the main absorption site.

Specific examples of the pH-dependent polymer base can include the following (1) to (3):

(1) methacrylic acid copolymers, wherein the methacrylic acid copolymers mean copolymers of two or more monomer species selected from the group consisting of methacrylic acid, methacrylic acid ester, acrylic acid, and acrylic acid ester and are not limited by the combination of the monomers, the number of the monomers used, etc.;
(2) hydroxypropyl methylcellulose acetate succinate; and
(3) carboxymethylethyl cellulose.

The "pH-dependent polymer base (B)" of the present invention is preferably the methacrylic acid copolymer (1) and hydroxypropyl methylcellulose acetate succinate (2) described above; more preferably hydroxypropyl methylcellulose acetate succinate (2).
The methacrylic acid copolymer (1) is preferably a methacrylic acid-methyl methacrylic acid copolymer, a (ethyl acrylate-methyl methacrylate-trimethylammonium ethyl methacrylate chloride) copolymer, a (methacrylic acid-ethyl acrylate) copolymer, a (methacrylic acid-methyl methacrylate) copolymer, more preferably a methacrylic acid-methyl methacrylic acid copolymer. Specific examples of the methacrylic acid-methyl methacrylic acid copolymer can preferably include EUDRAGIT, which is commercially available as EUDRAGIT L100-55 and EUDRAGIT L100.
Hydroxypropyl methylcellulose acetate succinate (HPMCAS) (2) can be purchased from Shin-Etsu Chemical Co., Ltd. as AQOAT (trade name), for example. The available grades of HPMCAS are LF, MF, HF, LG, MG, and HG, and grades MF and HF are preferable.
Moreover, the preferable particle size of the pH-dependent polymer base of the present invention is preferably $D_{50}$ of 100 or smaller, more preferably $D_{50}$ of 50 or smaller, even more preferably $D_{50}$ of 10 or smaller. Moreover, the preferable particle size of hydroxypropyl methylcellulose acetate succinate as the pH-dependent polymer base of the present invention is preferably $D_{50}$ of 40 or smaller, more preferably $D_{50}$ of 20 or smaller, even more preferably $D_{50}$ of 10 or smaller, particularly preferably $D_{50}$ of 5 or smaller.
The content of the pH-dependent polymer base (B) in the matrix pellet preparation of the present invention is in the range of preferably 5 to 90%, more preferably 50 to 80%.

A feature of the present invention is that a plasticizer (C) is further contained therein in addition to the pharmacologically active drug (A) and the pH-dependent polymer base (B).

As the plasticizer (C) according to the present invention, a plasticizer used as an acceptable additive for pharmaceuticals can be used. The plasticizer (C) is preferably citric acid tri(C1-C6 alkyl) ester or glycerin mono-organic acid ester, more preferably triethyl citrate and glycerin monoacetic acid ester, particularly preferably triethyl citrate. The content of the plasticizer (C) in the pellet preparation of the present invention is in the range of preferably 1 to 30%, more preferably 3 to 15%.

A feature of the sustained-release matrix pellet preparation of the present invention is that a low-melting-point oil or fat (D) is further contained therein in addition to the pharmacologically active drug (A), the pH-dependent polymer base (B), and the plasticizer (C).
Examples of the low-melting-point oil or fat (D) according to the present invention can include glycerin monostearate and polyethylene glycol. Glycerin monostearate and polyethylene glycol are preferred with polyethylene glycol being more preferred.

The polyethylene glycol is preferably polyethylene glycol having an average molecular weight in the range of 4000 to 12000, more preferably 7000 to 10500. Moreover, more specifically, the polyethylene glycol is preferably polyethylene glycol 6000 (CAS No.: 25322-68-3). Polyethylene glycol 6000 is Macrogol 6000 ($HOCH_2(CH_2OCH_2)_nCH_2OH$; n = 165 to 210) under the generic name and is commercially available as Macrogol 6000 (ADEKA Corp., etc.) or PEG6000 (Dai-Ichi Kogyo Seiyaku Co., Ltd.). The content of the low-melting-point oil or fat (D) in the pellet preparation of the present invention is in the range of preferably 0.1 to 20%, more preferably 1 to 6%.

The sustained-release matrix pellet preparation of the present invention may further contain one or two or more components selected from a binder and an organic acid.

The binder according to the present invention is preferably a cellulose derivative. Examples of the cellulose derivative can include hypromellose (HPMC: hydroxypropyl methylcellulose), hydroxypropyl cellulose (HPC), ethyl cellulose, and methyl cellulose. Hypromellose and hydroxypropyl cellulose are preferred, with hydroxypropyl cellulose being more preferred.
When hydroxypropyl cellulose is used, no particular limitation is imposed on its particle size used. For example, a commercially available fine powder (100-mesh sieve passing rate of 99%, average particle size: 150 microns) can be used. Hydroxypropyl cellulose (HPC) having a viscosity corresponding to grade L, SL, SSL, or the like may be used, and grade L (6.0 to 10 mPa·s) is preferred. When hydroxypropyl cellulose is used as a binding solution, this hydroxypropyl cellulose can usually be dissolved in water or an organic solvent such as an alcohol and used as a solution. The content of the binder in the pellet preparation of the present invention is in the range of preferably 0 to 10%, more preferably 0 to 2%.

The organic acid according to the present specification is preferably fumaric acid, succinic acid, alginic acid, adipic acid, citric acid, L-aspartic acid, malonic acid, maleic acid, DL-malic acid, or tartaric acid, more preferably fumaric acid or alginic acid, particularly preferably fumaric acid. The content of the organic acid in the pellets preparation of the present invention is in the range of preferably 0 to 40%, more preferably 0 to 20%.

The sustained-release matrix pellet preparation contains the pharmacologically active drug (A), the pH-dependent polymer base (B), the plasticizer (C), and the low-melting-point oil or fat (D) and may further contain a lubricant, a coloring agent, a polishing agent, etc., so long as the effects of the present invention are not impaired.

Examples of the lubricant include cocoa fat, carnauba wax, hydrous silicon dioxide, dry aluminum hydroxide gel, glycerin fatty acid ester, magnesium silicate, light anhydrous silicic acid, hardened oil, synthetic aluminum silicate,

white beeswax, magnesium oxide, sodium potassium tartrate, sucrose fatty acid ester, stearic acid, calcium stearate, magnesium stearate, stearyl alcohol, polyoxyl 40 stearate, cetanol, soybean hardened oil, gelatin, talc, magnesium carbonate, precipitated calcium carbonate, corn starch, potato starch, stearyl sodium fumarate, beeswax, magnesium metasilicate aluminate, sodium laurate, and magnesium sulfate.

Examples of the coloring agent can include yellow iron sesquioxide, iron sesquioxide, titanium oxide, orange essence, brown iron oxide, β-carotene, black iron oxide, food blue No. 1, food blue No. 2, food red No. 2, food red No. 3, food red No. 102, food yellow No. 4, and food yellow No. 5.

Examples of the polishing agent include carnauba wax, hardened oil, a polyvinyl acetate resin, white beeswax, titanium oxide, stearic acid, calcium stearate, polyoxyl 40 stearate, magnesium stearate, purified shellac, purified paraffin/carnauba wax mixture, cetanol, talc, colored silver foil, white shellac, paraffin, povidone, Macrogol 1500, Macrogol 4000, Macrogol 6000, beeswax, glycerin monostearate, and rosin.

No particular limitation is imposed on the particle size of the sustained-release matrix pellet preparation of the present invention, so long as the pellets preparation can be orally administered to a subject. However, matrix pellets are more preferred. Moreover, this sustained-release matrix pellet preparation is preferably encapsulated and used as a capsule.

The sustained-release matrix pellet preparation of the present invention may be produced through widely known production methods. In one exemplified procedure, the pharmaceutical composition of the present invention can be prepared through mixing the pharmacologically active drug (A), the pH-dependent polymer base (B), the plasticizer (C), and the low-melting-point oil or fat (D), and optional additives such as one or two or more components selected from a binder and an organic acid, a disintegrant, a binder, a fluidizing agent, a lubricant, a coloring agent, and a polishing agent, and the mixture can be processed through, for example, a method of producing pellet preparations generally used.

The present invention also relates to a method for producing the sustained-release pellet pharmaceutical composition described above. Hereinafter, the production method will be described in detail.
The sustained-release pellet pharmaceutical composition for oral administration of the present invention can be produced by the following steps (E) to (H) :

(E) mixing a pharmacologically active drug, a pH-dependent polymer base, and an oil or fat to prepare a [mixture];
(F) adding and stirring a plasticizer, an aqueous binder solution, and distilled water to prepare a [dispersion];
(G): producing a [kneaded product] from the [powder mixture] and the [dispersion]; and
(H) extrusion-granulating the [kneaded product] to produce [pellets].

In this context, in the aforementioned step (G), the pH-dependent polymer base is preferably hydroxypropyl methylcellulose acetate succinate, and the plasticizer is preferably triethyl citrate. In this step, the plasticizer is preferably used in the range of 5 to 30% by weight with respect to the pH-dependent polymer base.
The aforementioned step (H) is the step of extrusion-granulating the [kneaded product] to produce [pellets]. In this context, the [kneaded product] after extrusion granulation may be subjected to size selection operation using a size selector or the like. [Pellets] can be produced from the size-selected particles by annealing (heat treatment) using a usual drier. In this context, examples of the drier can include fluidized-bed granulation driers and shelf driers. Moreover, the temperature of annealing (heat treatment) and the treatment time can be appropriately adjusted according to the drug used, etc. Specifically, the annealing (heat treatment) is preferably carried out at an internal temperature of an apparatus in the range of 40 to 90°C for a duration in the range of 15 minutes to 2 hours, but the temperature of annealing (heat treatment) and the treatment time are not particularly limited to these ranges by any means.
Moreover, the particle sizes of the obtained [pellets] can be adjusted to an appropriate size using a sieve.

The proportion of the pharmacologically active drug (A) according to the present invention contained in the pharmaceutical composition of the present invention is usually preferably 0.1 to 50% by weight, more preferably 10 to 25% by weight, in terms of the free form of the pharmacologically active drug. Particularly, the dosage form of the pharmaceutical composition of the present invention is preferably a capsule in which pellets are encapsulated. In this case, when the drug used is, for example, compound (1), the content of the pharmacologically active drug contained per capsule is usually 0.5 to 500 mg, preferably 1 to 100 mg, more preferably 5 to 75 mg, particularly preferably 15 to 60 mg, in terms of its free form.

Hereinafter, another embodiment of the present invention will be described.

The sustained-release preparation of the present invention is a sustained-release preparation obtained by mixing of (A) a pharmacologically active drug, (B) hydroxypropyl methylcellulose acetate succinate, (C) a plasticizer, and (D) polyethylene glycol followed by extrusion granulation.

A feature of the preparation of the present invention is that it is obtained by mixing of the components (A), (B), (C), and (D) followed by extrusion granulation. The present invention does not encompass, for example, a preparation in which a preparation obtained by mixing of the components (A), (C), and (D) followed by extrusion granulation is provided with coating containing the component (B). However, the present invention encompasses, for example, a

preparation in which a preparation obtained by mixing of the components (A), (B), (C), and (D) followed by extrusion granulation is provided with coating containing the component (B).

Examples of the "pharmacologically active drug" used as the component (A) in the preparation of the present invention can include the compounds described above. Also, the "pharmacologically active drug" may be a prodrug that can be converted to the pharmacologically active drug in vivo. The content of the component (A) in the preparation of the present invention is preferably 0.1 to 60% by weight, more preferably 1 to 50% by weight, even more preferably 5 to 35% by weight.

Examples of the "hydroxypropyl methylcellulose acetate succinate (HPMCAS)" used as the component (B) in the preparation of the present invention can include the substances described above. The grade of HPMCAS is preferably LF, MF, or HF, more preferably MF or HF.

Moreover, the HPMCAS used as the component (B) in the preparation of the present invention has an average particle size ($D_{50}$) of preferably 50 μm or smaller, more preferably 20 μm or smaller, even more preferably 10 μm or smaller, particularly preferably 5 μm or smaller.

In the present specification, the term "$D_{50}$" refers to a particle size corresponding to the median value of a cumulative distribution curve determined using a laser diffraction-type meter HELOS (Japan Laser Corp.), i.e., a median size. Also, in the present specification, the term "$D_{90}$" refers to a particle size corresponding to 90% of the cumulative distribution curve determined using the HELOS. For example, $D_{90}$ of 20 μm means that 90% of the measured powders have a particle size of 20 μm or smaller and the remaining 10% have a particle size larger than 20 μm.

The content of the component (B) in the preparation of the present invention is preferably 40 to 90% by weight, more preferably 50 to 80% by weight, even more preferably 60 to 75% by weight.

Examples of the "plasticizer" used as the component (C) in the preparation of the present invention can include the substances described above. Triethyl citrate is preferred. The content of the component (C) in the preparation of the present invention is preferably 5 to 30% by weight.

Examples of the "polyethylene glycol (PEG)" used as the component (D) in the preparation of the present invention can include the substances described above. PEG having an average molecular weight of 4000 to 12000 is preferred, and PEG having an average molecular weight of 7000 to 10500 is more preferred. Examples of PEG having such a molecular weight can include polyethylene glycol 6000 (CAS No.: 25322-68-3). The content of the component (D) in the preparation of the present invention is preferably 1 to 10% by weight, more preferably 2 to 8% by weight, even more preferably 3 to 6% by weight.

In addition to the components (A) to (D), the preparation of the present invention may further contain a binder and/or an organic acid. Examples of the binder can include the substance described above. Hydroxypropyl cellulose or hydroxypropyl methylcellulose is preferred with hydroxypropyl cellulose being more preferred. Examples of the organic acid can include the substances described above. Fumaric acid or alginic acid is preferred with fumaric acid being more preferred. When the preparation of the present invention contains a binder and/or an organic acid, the content of the binder in the preparation is preferably 0.1 to 10% by weight, more preferably 0.5 to 2% by weight and the content of the organic acid in the preparation is preferably 0.1 to 40% by weight, more preferably 1 to 20% by weight.

The preparation of the present invention may further contain additives such as the lubricant, the coloring agent, and the polishing agent, so long as the effects of the present invention are not impaired.

The preparation of the present invention is produced by mixing of the components (A) to (D) followed by extrusion granulation. More specifically, the components (A), (B), (C), and (D) are mixed, and a solution containing a binder or distilled water is added to the mixture. Then, the resulting mixture can be kneaded and extrusion-granulated to produce the preparation of the present invention. The obtained preparation may be compression-molded or coated, if necessary. The mixing, kneading, extrusion granulation, compression molding, coating, and the like can be performed using methods well known in the art. When the preparation of the present invention contains additional additives, these additives may be added thereto in any of mixing, kneading, compression, and coating steps.

In the preparation thus obtained, the components are preferably formulated in a matrix form.

The preparation of the present invention thus obtained has a favorable tablet strength that prevents dose dumping in an acidic solution, and has favorable dissolution properties in a neutral solution, as described in Advantageous Effects of the Invention above. Thus, the preparation of the present invention is effective for maintenance of prolonged dissolution of the pharmacologically active drug contained therein from the duodenum through the small intestine to the lower gastrointestinal tract.

Examples

Next, the present invention will be described in detail with reference to Examples. However, the present invention is not intended to be limited to these by any means.

Abbreviations used in Examples are as follows:

HPMCAS-HF1: hydroxypropyl methylcellulose acetate succinate grade LF ($D_{50}$: 5 μm, $D_{90}$: 11 μm, the content of succinoyl group being 7.3 to 8.0%) (manufactured by Shin-Etsu Chemical Co., Ltd.)
HPMCAS-HF2: hydroxypropyl methylcellulose acetate succinate grade LG ($D_{50}$: 5 μm, $D_{90}$: 11 μm, the content of succinoyl group being 4.0 to 7.2%) (manufactured by Shin-Etsu Chemical Co., Ltd.)
HPMCAS-MF: hydroxypropyl methylcellulose acetate succinate grade MF ($D_{50}$: 5 μm, $D_{90}$: 11 μm) (manufactured by Shin-Etsu Chemical Co., Ltd.)
PEG 6000: polyethylene glycol 6000
HPC-L: hydroxypropyl cellulose grade L (manufactured by Nippon Soda Co., Ltd.)

$D_{50}$ and $D_{90}$ were measured at a dispersion pressure of 3 bar in a measurement range of R4 using a laser diffraction-type particle size distribution meter HELOS&RODOS (Japan Laser Corp.).
A collection rate was calculated for each preparation according to the following equation:

(Weight of pellets having particular particle size distribution, of pellets obtained by extrusion granulation) / (weight of kneaded product) × 100.

A collection rate of 80% or more was evaluated as being good (O) while a collection rate less than 80% was evaluated as being poor (x).
Tests on dissolution properties in an acidic or neutral solution were conducted as follows:

(Dissolution test in acidic solution)

The dissolution test was conducted by the paddle method at rotation rates of 50 rpm at 37 ± 0.5°C in 0.01 N hydrochloric acid (900 mL), and the time-dependent average percentage dissolution of a drug in the dissolution medium was calculated.

(Dissolution test in neutral solution)

The dissolution test was conducted by the paddle method at a rotation rate of 50 rpm at 37 ± 0.5°C in phosphate buffer (pH 6.8, 900 mL), and the time-dependent average percentage dissolution of a drug in the dissolution medium was calculated.

(Example 1)

For formulations 1 to 4 shown in Table 1, pulverized compound (1a) and HPMCAS-HF1 were mixed for 5 minutes in a vinyl bag. Then, 40 mL of 5% aqueous HPC-L solution containing triethyl citrate dispersed therein and 90 mL of purified water were added to the obtained mixture and kneaded for 5 minutes using a high-speed mixer. The obtained kneaded product was extrusion-granulated in an extrusion granulator (Domegran, screen diameter: 1.0 mmϕ) and annealed in a shelf drier (80°C, 2 hr). The pellets thus annealed were sifted through Nos. 14 and 18 sieves, and pellets that passed through No. 14 sieve and remained on No. 18 sieve were collected to prepare each preparation. The collection rate for each preparation is shown in Table 2, and results of the dissolution test in an acidic solution are shown in Figure 1.
[Table 1]

| | Content (mg) | | | |
|---|---|---|---|---|
| | Formulation 1 | Formulation 2 | Formulation 3 | Formulation 4 |
| Compound (1a)<br>(Compound (1a-1)) | 36.4<br>(30.0) | 36.4<br>(30.0) | 36.4<br>(30.0) | 36.4<br>(30.0) |
| HPMCAS-RF1 | 142.3 | 142.3 | 142.3 | 142.3 |
| Triethyl citrate | 21.3 | 21.3 | 21.3 | 21.3 |

(continued)

| | Content (mg) | | | |
|---|---|---|---|---|
| | Formulation 1 | Formulation 2 | Formulation 3 | Formulation 4 |
| HPC-L | 6.0 | 2.0 | 2.0 | 2.0 |
| Pregelatinized starch (PC-10) | - | 10.0 | - | - |
| Glycerin monostearate | - | - | 10.0 | - |
| PEG6000 | - | - | - | 10.0 |
| Total | 206.0 | 212.0 | 212.0 | 212.0 |

[Table 2]

| Formulation | Formulation 1 | Formulation 2 | Formulations 3 | Formulation 4 |
|---|---|---|---|---|
| Collection rate for preparation | × | ○ | ○ | ○ |

<Test results>

Formulations having a favorable collection rate were formulations 2 to 4 supplemented with pregelatinized starch, glycerin monostearate, or PEG6000. However, as shown in Figure 1, the preparation of formulation 2 supplemented with pregelatinized starch exhibited a rapid dissolution rate of the drug in the acidic solution compared with the preparations of formulations 1, 3, and 4.

(Example 2)

For formulations 5 to 8 shown in Table 3, pellets were prepared in the same way as in Example 1. The obtained pellets were sifted through Nos. 14 and 30 sieves, and pellets that passed through No. 14 sieve and remained on No. 30 sieve were collected to prepare each preparation. The collection rate for each preparation is shown in Table 4, and results of the dissolution test in an acidic solution are shown in Figure 2.

[Table 3]

| | Content (mq) | | | |
|---|---|---|---|---|
| | Formulation 5 | Formulation 6 | Formulation 7 | Formulation 8 |
| Compound (1b)<br>(Compound (1b-1)) | 80.8<br>(60.0) | 80.8<br>(60.0) | 80.8<br>(60.0) | 80.8<br>(60.0) |
| HPMCAS-HF1 | 199.3 | 199.3 | 199.3 | 199.3 |
| Triethyl citrate | 19.9 | 19.9 | 19.9 | 19.9 |
| HPC-L | 3.0 | 2.0 | 2.0 | 2.0 |
| Pregelatinized starch(PC-10) | - | 10.0 | - | - |
| Glycerin monostearate | - | - | 10.0 | - |
| PEG6000 | - | - | - | 10.0 |
| Total | 303.0 | 312.0 | 312.0 | 312.0 |

[Table 4]

| Formulation | Formulation 5 | Formulation 6 | Formulation 7 | Formulation 8 |
|---|---|---|---|---|
| Collection rate for preparation | × | ○ | ○ | ○ |

<Test results>

Formulations having a favorable collection rate were formulations 6 to 8 supplemented with pregelatinized starch, glycerin monostearate, or PEG6000. However, as shown in Figure 2, the preparation of formulation 6 supplemented with pregelatinized starch exhibited a rapid dissolution rate of the drug in the acidic solution compared with the preparations of formulations 5, 7, and 8.

(Example 3)

For formulations 9 and 10 shown in Table 5, pellets were prepared in the same way as in Example 1. The obtained pellets were sifted through Nos. 14 and 18 sieves, and pellets that passed through No. 14 sieve and remained on No. 18 sieve were collected to prepare each preparation. The obtained preparation was placed in a vial and, after hermetical sealing of the vial, stored at 40°C, 50°C, or 60°C for 1 day or 1 week. The preparation was subjected to the dissolution test in a neutral solution immediately after the preparation and after the storage, and the results are shown in Figures 3 and 4.

[Table 5]

| | Content (mg) | |
|---|---|---|
| | Formulation 9 | Formulation 10 |
| Compound (1a)<br>(Compound (1a-1)) | 36.4<br>(30.0) | 36.4<br>(30.0) |
| HPMCAS-MF | 74.6 | 74.6 |
| HPMCAS-HF1 | 74.6 | 74.6 |
| Triethyl citrate | 22.4 | 22.4 |
| HPC-L | 2.0 | 2.0 |
| Glycerin monostearate | 10.0 | - |
| PEG6000 | - | 10.0 |
| Total | 220.0 | 220.0 |

<Test results>

As shown in Figure 3, the 1-day storage at 40°C or 50°C delayed drug dissolution from the preparation of formulation 9 supplemented with glycerin monostearate. Moreover, the 1-day storage at 60°C accelerated the dissolution behaviors. By contrast, as shown in Figure 4, little change was observed in the dissolution behaviors of the preparation of formulation 10 supplemented with PEG6000 even after the storage at 40°C, 50°C, or 60°C for 1 week.

(Example 4)

For formulations 7 and 8 shown in Table 3, each obtained preparation was placed in a vial and, after hermetical sealing of the vial, stored at 50°C for 3 days. The preparation was subjected to the dissolution test in a neutral solution immediately after the preparation and after the storage. As a result, the 3-day storage at 50°C delayed drug dissolution from the preparation of formulation 7 supplemented with glycerin monostearate. By contrast, little change was observed in the dissolution behaviors of the preparation of formulation 8 supplemented with PEG6000 even after the storage at 50°C for 3 days.

(Example 5)

For formulation 11 shown in Table 6, pellets were prepared in the same way as in Example 1. The obtained pellets were sifted through Nos. 14 and 30 sieves, and pellets that passed through No. 14 sieve and remained on No. 30 sieve were collected to prepare a preparation. The obtained preparation was subjected to the dissolution test in a neutral solution, and the results are shown in Figure 5.

[Table 6]

| | Content (mg) |
|---|---|
| | Formulation 11 |
| Compound (1a)<br>(Compound (1a-1)) | 36.4<br>(30.0) |
| HPMCAS-MF | 14.9 |
| HPMCAS-HF1 | 134.3 |
| Triethyl citrate | 22.4 |
| HPC-L | 2.0 |
| PEG6000 | 10.0 |
| Total | 220.0 |

<Test results>

As shown in Figure 5, prolonged dissolution behaviors of the drug were obtained in the neutral solution.

(Example 6)

For formulation 12 shown in Table 7, pellets were prepared in the same way as in Example 1 except that the screen diameter for extrusion granulation was changed to 1.2 mmϕ). The obtained pellets were sifted through Nos. 12 and 18 sieves, and pellets that passed through No. 12 sieve and remained on No. 18 sieve were collected to prepare a preparation. The obtained preparation was subjected to the dissolution test in a neutral solution, and the results are shown in Figure 6.

[Table 7]

| | Content (mg) |
|---|---|
| | Formulation 12 |
| Compound (1a)<br>(Compound (1a-1)) | 36.4<br>(30.0) |
| HPMCAS-MF | 74.6 |
| HPMCAS-HF1 | 74.6 |
| Triethyl citrate | 14.9 |
| Fumaric acid | 20.0 |
| HPC-L | 2.0 |
| PEG6000 | 10.0 |
| Total | 232.5 |

<Test results>

As shown in Figure 6, prolonged dissolution behaviors of the drug were obtained in the neutral solution.

(Example 7)

For formulations 13 and 14 shown in Table 8, pellets were prepared in the same way as in Example 1. The obtained pellets were sifted through Nos. 14 and 30 sieves, and pellets that passed through No. 14 sieve and remained on No. 30 sieve were collected to prepare each preparation. The obtained preparation was encapsulated into a hydroxypropyl methylcellulose capsule.

[Table 8]

| | Content (mg) | |
|---|---|---|
| | Formulation 13 | Formulation 14 |
| Compound (1a) (Compound (1a-1)) | 36.4 (30.0) | 36.4 (30.0) |
| HPMCAS-MF | 14.9 | - |
| HPMCAS-HF1 | 59.7 | - |
| HPMCAS-HF2 | 74.6 | 149.2 |
| Triethyl citrate | 22.4 | 22.4 |
| HPC-L | 2.0 | 2.0 |
| PEG6000 | 10.0 | 10.0 |
| Total | 220.0 | 220.0 |

<Test results>

As a result of administering these capsules to humans, these capsules exhibited lower Cmax and a higher blood concentration after 24 hours than those of a preparation having the same amount of the drug (compound (1a)) dissolved in water, demonstrating that these capsules exhibited favorable profiles as sustained-release preparations.

Industrial Applicability

The present invention can be used in the production of a sustained-release matrix pellet preparation for oral administration containing a pharmacologically active drug such as compound (1).

**Claims**

1. A sustained-release preparation obtained by mixing of

    (A) a pharmacologically active drug,
    (B) hydroxypropyl methylcellulose acetate succinate,
    (C) a plasticizer, and
    (D) polyethylene glycol

    followed by extrusion granulation.

2. The preparation according to claim 1, wherein the content of the component (B) in the preparation is 40 to 90% by weight.

3. The preparation according to claim 1, wherein the content of the component (B) in the preparation is 50 to 80% by weight.

4. The preparation according to any one of claims 1 to 3, wherein the component (C) is triethyl citrate.

5. The preparation according to any one of claims 1 to 4, wherein the content of the component (C) in the preparation is 5 to 30% by weight.

6. The preparation according to any one of claims 1 to 5, wherein the component (D) is polyethylene glycol having an average molecular weight in the range of 4000 to 12000.

7. The preparation according to any one of claims 1 to 5, wherein the component (D) is polyethylene glycol having an average molecular weight in the range of 7000 to 10500.

8. The preparation according to any one of claims 1 to 5, wherein the component (D) is polyethylene glycol 6000.

9. The preparation according to any one of claims 1 to 8, wherein the content of the component (D) in the preparation is 1 to 10% by weight.

10. The preparation according to any one of claims 1 to 8, wherein the content of the component (D) in the preparation is 2 to 8% by weight.

11. The preparation according to any one of claims 1 to 10, further containing a binder and/or an organic acid.

12. The preparation according to claim 11, wherein the binder is hydroxypropyl cellulose.

13. The preparation according to claim 11 or 12, wherein the organic acid is fumaric acid or alginic acid.

14. The preparation according to claim 11 or 12, wherein the organic acid is fumaric acid.

15. The preparation according to any one of claims 1 to 14, wherein the component (A) is a basic drug.

16. The preparation according to any one of claims 1 to 14, wherein the component (A) is a compound selected from the group consisting of

(+)-1-(carbazol-4-yloxy)-3-[[2-(o-methoxyphenoxy)ethyl]amino]-2-propanol,
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide, and
$N^1$-(5-chloropyridin-2-yl)-$N^2$-[(1S,2R,4S)-2-{[(5-methyl-4,5,6,7-tetrahydrothiazalo[5,4-c]pyridin-2-yl)carbonyl]amino}-4-([1,3,4]oxadiazol-2-yl)cyclohexyl]ethanediamide

or a pharmacologically acceptable salt thereof, or a hydrate thereof.

17. The preparation according to any one of claims 1 to 16, wherein the content of the component (A) in the preparation is 0.1 to 50% by weight.

[Figure 1]

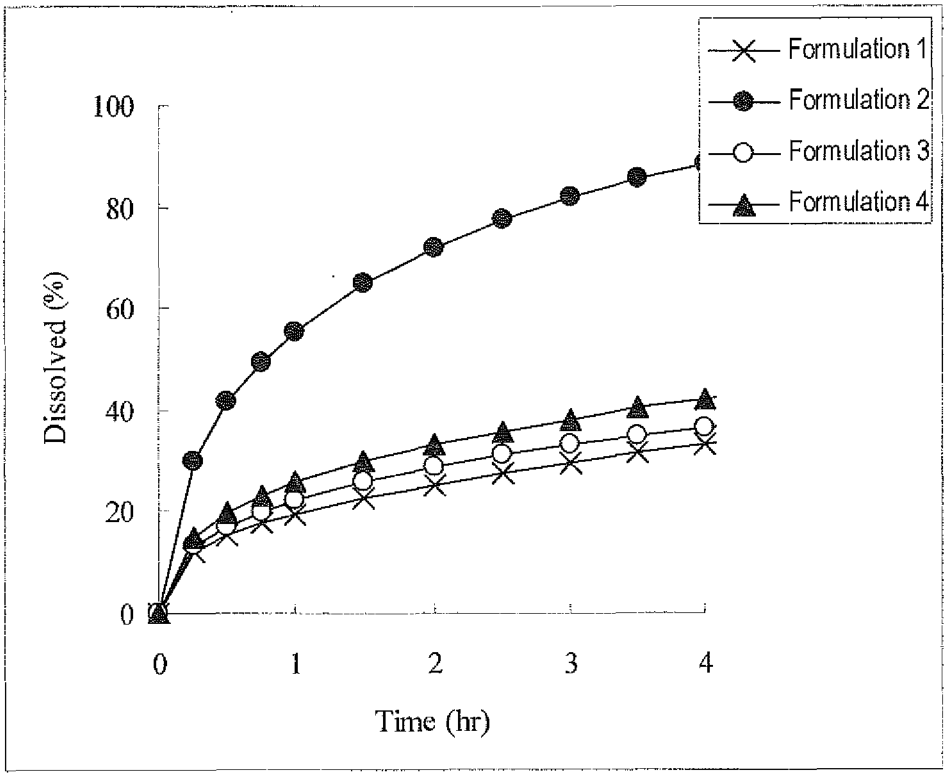
Formulation 1
Formulation 2
Formulation 3
Formulation 4
Dissolved (%)
100
80
60
40
20
0
0
1
2
3
4
Time (hr)

[Figure 2]

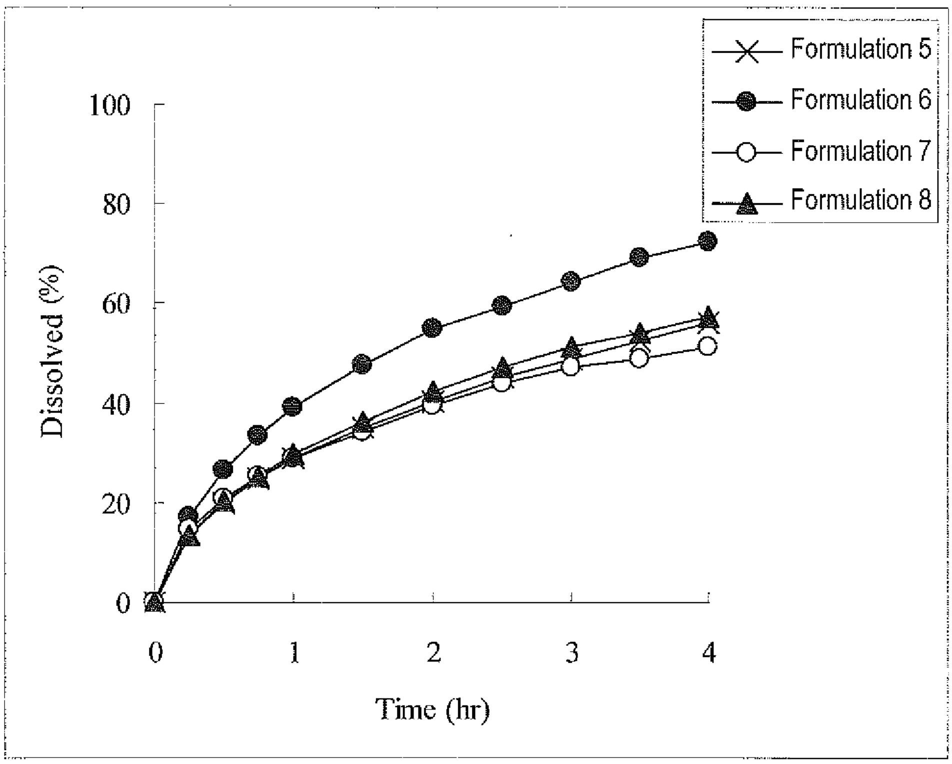
Formulation 5
Formulation 6
Formulation 7
Formulation 8
100
80
60
40
20
0
Dissolved (%)
0
1
2
3
4
Time (hr)

[Figure 3]

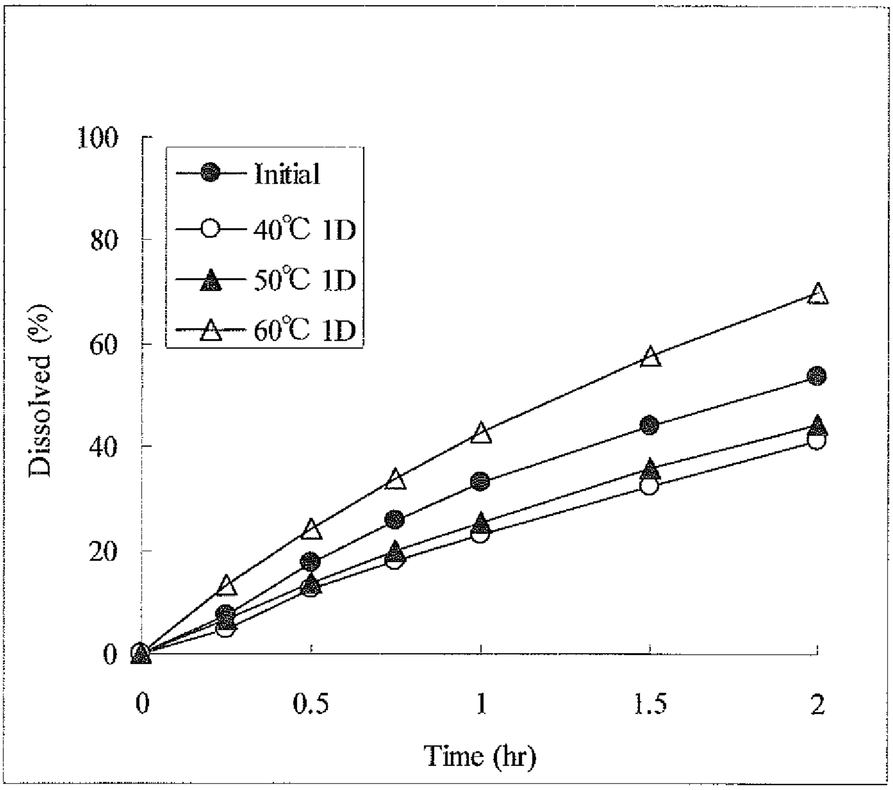
100
80
60
40
20
0
Dissolved (%)
0
0.5
1
1.5
2
Time (hr)
Initial
40℃ 1D
50℃ 1D
60℃ 1D

[Figure 4]

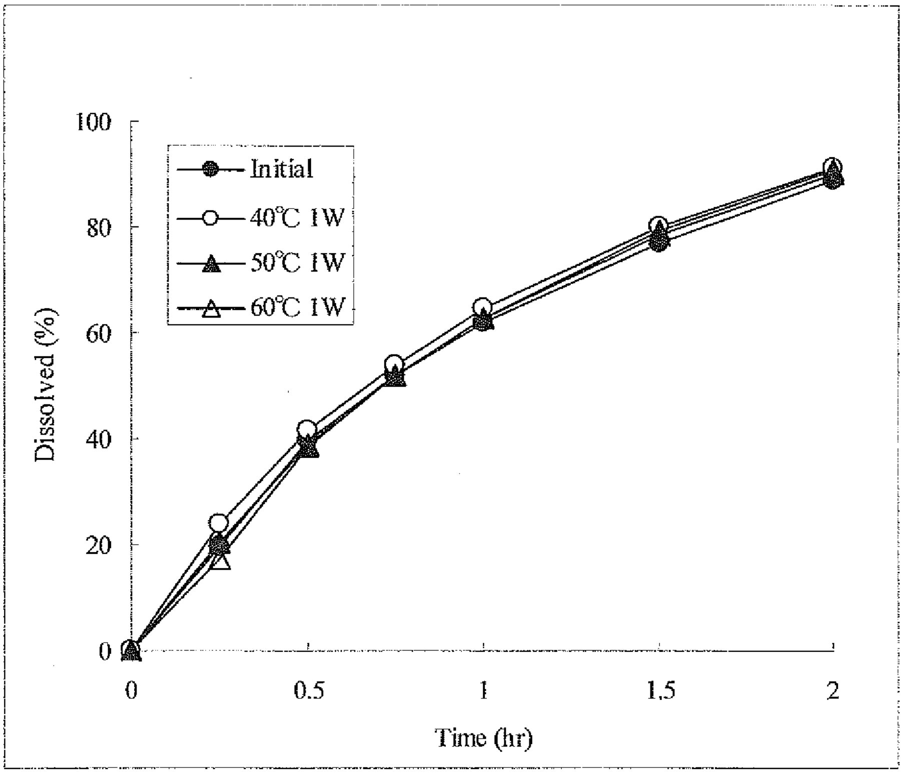
Initial
40℃ 1W
50℃ 1W
60℃ 1W
Dissolved (%)
100
80
60
40
20
0
0
0.5
1
1.5
2
Time (hr)

[Figure 5]

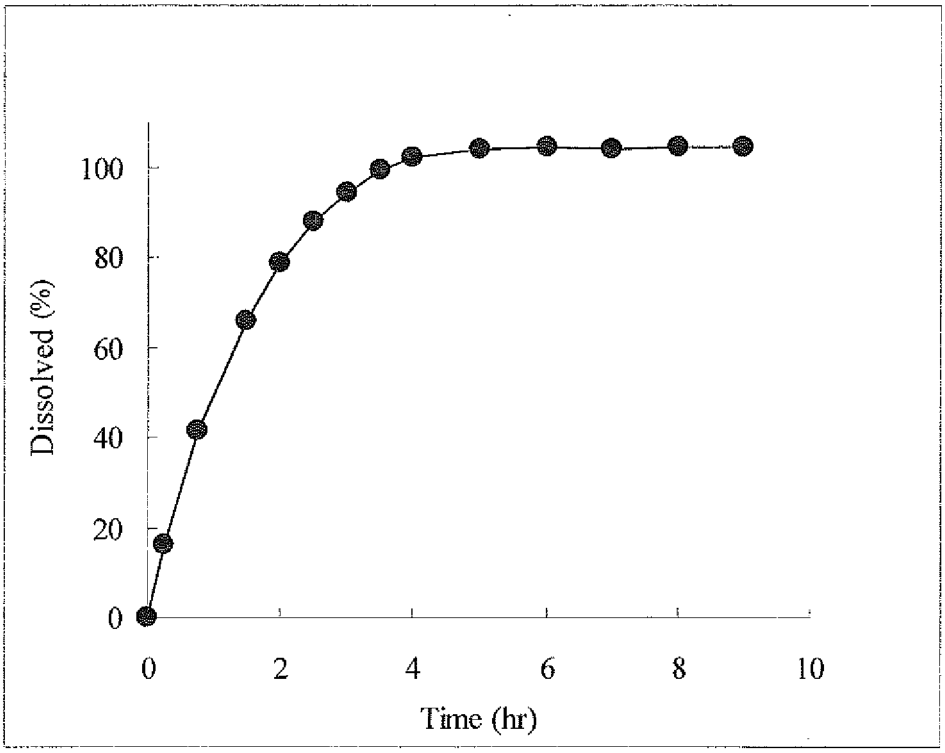


[Figure 6]

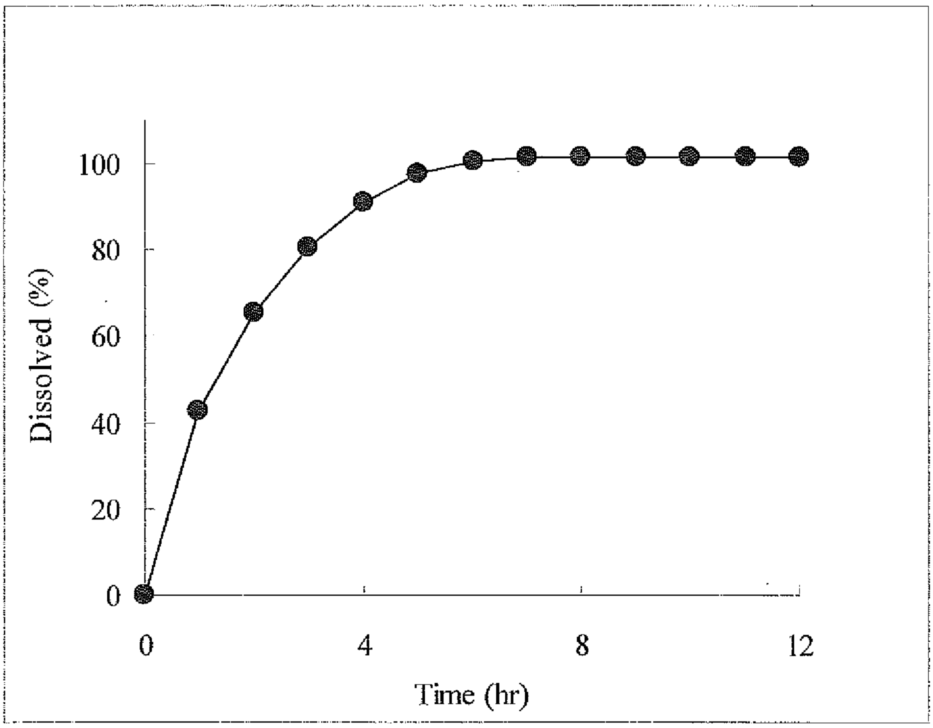

## INTERNATIONAL SEARCH REPORT

International application No.
PCT/JP2011/053643

A. CLASSIFICATION OF SUBJECT MATTER
*A61K47/34*(2006.01)i, *A61K9/16*(2006.01)i, *A61K31/403*(2006.01)i, *A61K31/4745*(2006.01)i, *A61K47/12*(2006.01)i, *A61K47/14*(2006.01)i, *A61K47/38*(2006.01)i, *A61P7/02*(2006.01)i

According to International Patent Classification (IPC) or to both national classification and IPC

B. FIELDS SEARCHED

Minimum documentation searched (classification system followed by classification symbols)
A61K47/34, A61K9/16, A61K31/403, A61K31/4745, A61K47/12, A61K47/14, A61K47/38, A61P7/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2006/070781 A1 (Eisai Co., Ltd.),<br>06 July 2006 (06.07.2006),<br>claims; paragraphs [0046], [0054]; examples 12, 13<br>& US 2006/0280789 A1 & US 2007/0129402 A1<br>& EP 1832298 A1 | 1-5,9-15,17<br>4,6-8,16 |
| X<br><br>Y | JP 2007-537183 A (Bayer Healthcare AG.),<br>20 December 2007 (20.12.2007),<br>claims; paragraphs [0036], [0038], [0040], [0043], [0055], [0066]; example 9<br>& US 2008/0187588 A1 & WO 2005/110420 A1 | 1-3,5,9-15, 17<br><br>4,6-8,16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
30 March, 2011 (30.03.11)

Date of mailing of the international search report
12 April, 2011 (12.04.11)

Name and mailing address of the ISA/
Japanese Patent Office

Authorized officer

Facsimile No.

Telephone No.

Form PCT/ISA/210 (second sheet) (July 2009)

INTERNATIONAL SEARCH REPORT

International application No.
PCT/JP2011/053643

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2007-537175 A (Bayer Healthcare AG.),<br>20 December 2007 (20.12.2007),<br>claims; paragraphs [0036], [0038], [0040], [0043], [0055], [0066]; examples 9, 29, 30<br>& US 2008/0268046 A1 & WO 2005/110419 A1 | 1-3,11-15,17<br>4,6-8,16 |
| Y | JP 63-107933 A (Chugai Pharmaceutical Co., Ltd.),<br>12 May 1988 (12.05.1988),<br>example 2<br>& US 5547943 A & DE 3714159 A<br>& FR 2598084 A | 6-8 |
| Y | JP 2004-26750 A (Nippon Shinyaku Co., Ltd.),<br>29 January 2004 (29.01.2004),<br>example 3<br>(Family: none) | 6-8 |
| Y | WO 2005/000312 A1 (Otsuka Pharmaceutical Co., Ltd.),<br>06 January 2005 (06.01.2005),<br>page 18, lines 11 to 23<br>& US 2007/0098843 A1 & EP 1640006 A1 | 6-8 |
| Y | JP 2001-522794 A (Boehringer Mannheim Pharmaceuticals Corp. - Smithkline Beckman Corp. Ltd. Partnership No.1),<br>20 November 2001 (20.11.2001),<br>claims<br>& US 2002/0182256 A1 & US 2004/0220250 A1<br>& EP 1030651 A & WO 1999/024017 A1 | 16 |
| Y | JP 2001-513752 A (Roche Diagnostics GmbH),<br>04 September 2001 (04.09.2001),<br>claims<br>& US 6224909 B1 & US 2001/0004458 A1<br>& EP 925060 A & WO 1998/010754 A1 | 16 |
| Y | WO 2003/000657 A1 (Daiichi Pharmaceutical Co., Ltd.),<br>03 January 2003 (03.01.2003),<br>example 310<br>& US 2005/0119486 A1 & EP 1405852 A1 | 16 |
| Y | WO 2003/000680 A1 (Daiichi Pharmaceutical Co., Ltd.),<br>03 January 2003 (03.01.2003),<br>example 310<br>& US 2005/0245565 A1 & EP 1415992 A1 | 16 |

Form PCT/ISA/210 (continuation of second sheet) (July 2009)

**INTERNATIONAL SEARCH REPORT**

International application No. PCT/JP2011/053643

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2003/016302 A1 (Daiichi Pharmaceutical Co., Ltd.),<br>27 February 2003 (27.02.2003),<br>example 310<br>& US 2005/0020645 A1 | 16 |
| Y | WO 2004/058715 A1 (Daiichi Pharmaceutical Co., Ltd.),<br>15 July 2004 (15.07.2004),<br>example 385<br>& JP 2007-70369 A & JP 2008-138011 A<br>& JP 4109288 B & US 2006/0252837 A1<br>& US 2010/0099660 A & US 7576135 B2<br>& EP 1577301 A1 | 16 |
| A | JP 2001-524131 A (Sage Pharmaceuticals, Inc.),<br>27 November 2001 (27.11.2001),<br>page 15, lines 7 to 8<br>& US 2001/0006649 A1 & US 2003/0203018 A1<br>& US 2004/0197394 A1 & WO 1998/050019 A1 | 1-17 |

Form PCT/ISA/210 (continuation of second sheet) (July 2009)

**INTERNATIONAL SEARCH REPORT**

International application No.
PCT/JP2011/053643

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions in claims 1 - 4, 11 - 15, 17 have no special technical feature, since the inventions are described in the following documents 1 - 3 and cannot be considered to have novelty.

Document 1: WO 2006/070781
Document 2: JP 2007-537183
Document 3: JP 2007-537175

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006507216 A
- WO 2004518676 A
- WO 2004058715 A
- WO 03000657 A
- WO 03000680 A
- WO 03016302 A
- WO 2003000657 A
- WO 2003000680 A
- WO 2003016302 A


### Non-patent literature cited in the description


- Pre-publication copy, Proposed INN: List 101; Research and development pipeline. PFSB/ELD No. 1111-1. Yamanouchi Pharmaceutical Co Ltd. Company World Wide, 11 November 2010
- *WFO Drug Information,* 2004, vol. 18 (3), 260
- **SUSANNE R et al.** *J. Med. Chem.,* 2005, vol. 48, 5900-5908
- **D. KUBITZA et al.** Multiple dose escalation study investigating the pharmacodynamics, safety, and pharmacokinetic of Bay59-7939, an oral, direct Factor Xa inhibitor, in healthy male subjects. *Blood,* 2003, vol. 102
- *WFO Drug Information,* 2006, vol. 20 (1), 38
- **PINTO DJP ; ORWAT MJ ; LAM PYS et al.** Discovery of 1-(4-Methoxyphenyl)-7-oxo-6-(4-(2-oxopiperidin-1-yl)phenyl)-4,5,6,7-tetrahydro-1H-pyrazolo[3,4-c]pyridine-3-carboxamide (Apixaban, BMS-562247), a highly potent, selective, efficacious, and orally bioavailable inhibitor of blood coagulation factor Xa. *J. Med. Chem.,* 2007, vol. 50 (22), 5339-56
- **ZHANG P ; HUANG W ; ZHU BY.** *WFO Drug Information,* 2008, vol. 22 (3), 226-227
- Discovery of betrixaban (PRT054021), N-(5-chloropyridin-2-yl)-2-(4-(N,N-dimethylcarbamimidoyl)benzamido)-5-methoxybenzamide, a highly potent, selective, and orally efficacious factor Xa inhibitor. *Bioorg Med. Chem. Lett.,* 2009, vol. 19 (8), 2179-85
- **S. TAKEHANA et al.** *Japanese Journal of Pharmacology,* 2000, vol. 82 (1), 213
- **T. KAYAHARA et al.** *Japanese Journal of Pharmacology,* 2000, vol. 82 (1), 213
- Generating greater value from our products and pipeline. XVIIth Congress of the International Society for Thrombosis and Haemostasis. Aventis SA Company Presentation, 14 August 1999
- *WFO Drug Information,* 2002, vol. 16 (3), 257
- 226th National Meeting. American Chemical Society, 2003
- **J. R. PRUITT et al.** *J. Med. Chem.,* 2003, vol. 46, 5298-5313
- **S. YOUNG.** *Medicinal Chemistry-12th RSC-SCI Symposium,* 07 September 2003
- **M. WILEY et al.** *228th ACS National Meeting,* 22 August 2004, 252, 254